# EUROPEAN PATENT APPLICATION

(11) **EP 1 820 473 A1**
(43) Date of publication of application: **22.08.2007**
(21) Application number: 06110222.4
(22) Date of filing: 21.02.2006
(51) Int. Cl.: A61F 2/12, A61F 2/26

(54) **Refillable and re-inflatable implants**

(71) Applicant: Tytgadt, Didier, 9052 Gent (BE)
(72) Inventor: Tytgadt, Didier, 9052 Gent (BE)
(74) Representative: Van Reet, Joseph

(57) **Abstract**

A multi refillable and/or re-inflatable, flexible and/or extensible implant made in soft materials and based on a new concept and a new methodology for body implants, with various principles explained further, to allow multiple and/or repetitive transformation of body parts - as are breasts and penis for instance - which can be fully filled, emptied and refilled by people at home, without surgery and whereby the technical method allows multiple and repetitive transformation through an internal build up of a 2 or 3-dimensional grated membrane structure inside the shell of the implant, forming a multi cavity structure and whereby this grated membrane structure contains a fluid transition system for filling and emptying through permeability or small holes properly placed in the membranes to allow cavity connections, which bring the cavities of that grated structure in connection to a in/outlet system.

## Description

The present invention relates to soft volumetric body implants, for example for breast-implants & penis-implants.

### INTRODUCTION:

Actual breast implants have different disadvantages which we want to reduce or even resolve:
- For surgery the doctor needs to make a big section or cut or stab wound to introduce the breast implant. Our new design and structure allow reducing this stab wound or section or cut in combination with the new technical surgery instruments.
- After surgery and implantation, the patient regularly suffers from body tissues stiffening. Our new design, structure and flexibility of the implant allow reducing these stiffening effects.
- The existing lack of symmetry of the human bodies needs to adapt the implants in cases of required symmetry. Our new design, structure and flexibility of the implant allow reducing totally these volumetric adaptation problems during and after surgery by the patient.
- The potentiality for the patient to return to the initial situation is inexistent without new (major or minor) surgery. Our new design and concept of structure and flexibility of the implant allow reducing these disadvantages drastically and avoid new (major or minor) surgery and its effects.
- The wrinkle of the shell of the body implant can be avoided through the new design and concept of structure.

### DEFINITIONS:

- Membrane: very thin and flat double sided type of sheet, which takes no place when folded; it can be permeable!
- Grid: parallel lines in 2 directions (square or not square) when projected in a plane;
- Grid of membranes: grid where the lines becomes membranes in the 3rd dimension enclosing between each other the cavities to be filled up with the liquid. These cavities can be like long shafts. You can even add membranes in 3^{rd} dimension to form small cavities!
- Implant: Object, made in soft usual medical materials, which is conceived for insertion in the human body!
- Cavity: inner space between the membranes, inside the implant, that can have any shape and size related to the grid of membranes in 2 or 3 directions!
- Cavity structure: any 3 dimensional structures in between the membranes enclosing those cavities which can be made interconnected by little holes or through their own permeability for fluid transfer, as for instance a honeycomb structure, or a 3 dimensional grid structure.
- Tube: hollow cylindrical, oval or similar half oval (or any section allowing liquid flow through) and soft sheet material connecting at its 2 ends respectively the implant and a body exit through the skin
- Plug: closing system that can be cylindrical, conical or any shape fitting into the tube end and being formed of a (harder) material that fits into the shape of the tube end portion at the implant side or before the exit side. It can plug through simple pulling into one of the tube ends but it can plug by turning into a screw thread in this tube end, or it can expand or meet any liquid proof closing system. The plug needs to avoid any leakage of the implant.
- Valve: The plug can be replaced by any valve system satisfying the required characteristics of being used several times and closing the body implant securely! It can be commanded manually or wireless.

### FIGURES & DRAWINGS:

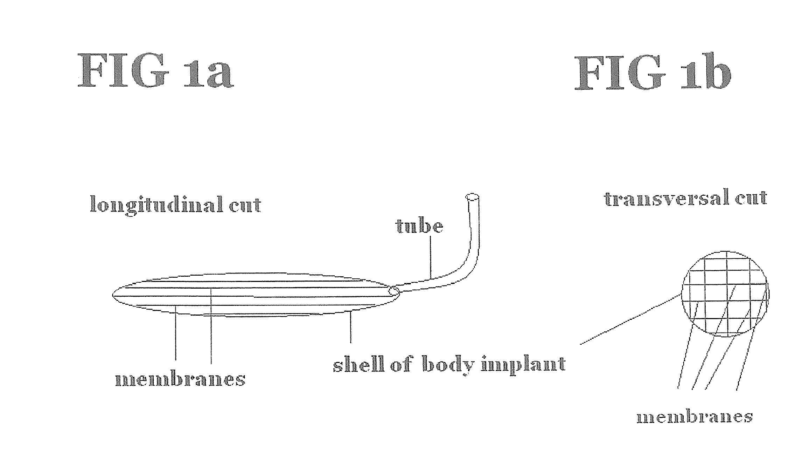

Figures : the shapes shown are not at scale and only illustrating the principles, therefore only 2 dimensional grid are drawn, no 3 dimensional grids which also are part of this patent request!
   - FIG 1 A: : sample of body implant for penis with 2-dimensional grid
   - FIG 1 B: : transversal section or cut of implant showing possible grid structure

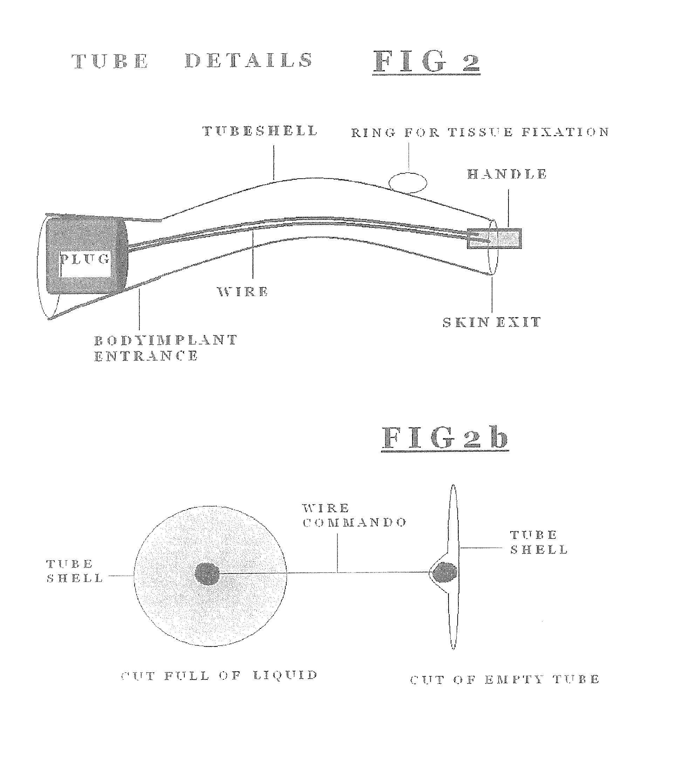

   - FIG 2A: : tube detail connecting the inlet of the body implant with the outlet through the skin
   - FIG 2B: : transversal (increased scale) section or cut B of tube with inside wired commando

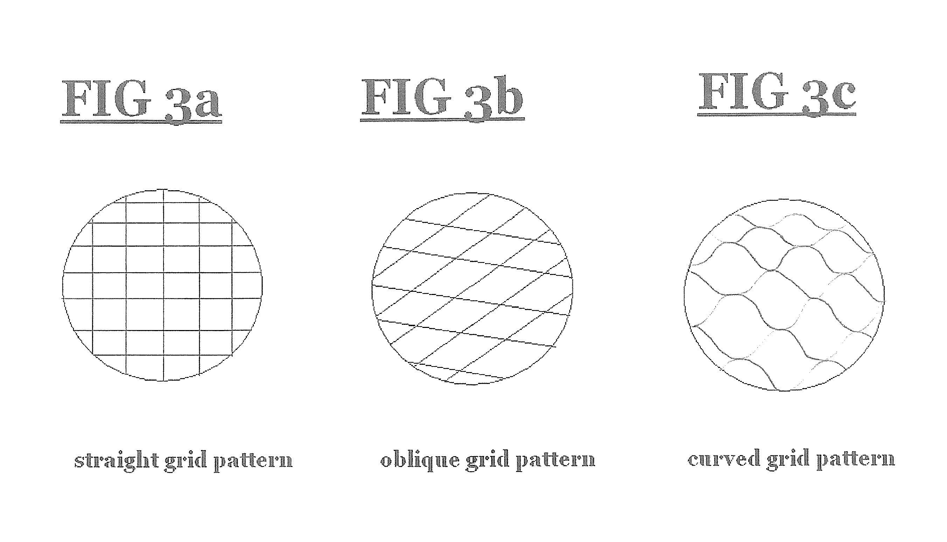

   - FIG 3A: : plan view of grid structure sample for membranes (straight angles)
   - FIG 3B: : plan view of grid structure sample for membranes (oblique angels)
   - FIG 3C: : plan view of grid structure sample for membranes (curved)

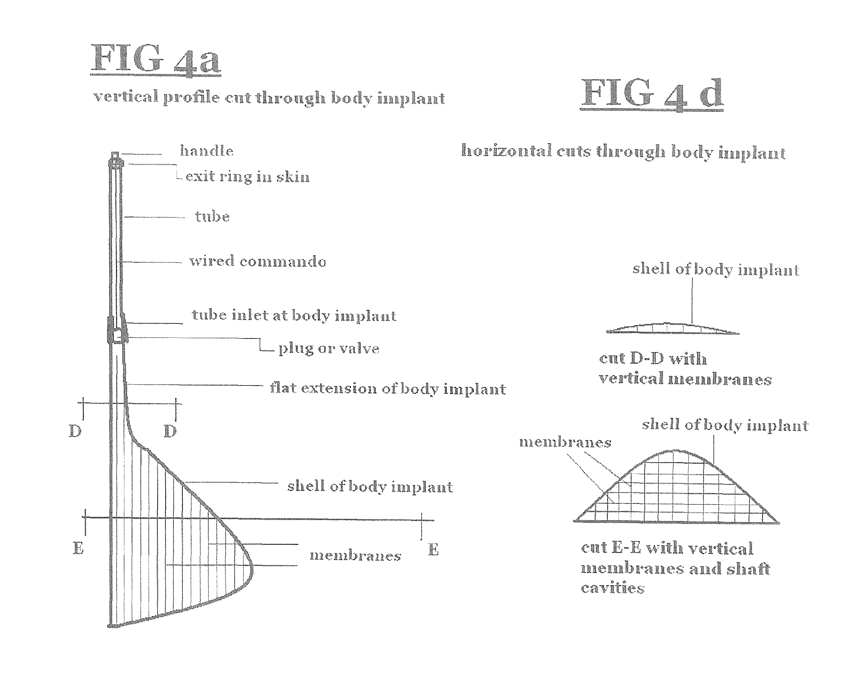

   - FIG 4A: : sample of transversal section or cut of body implant being a breast with upper side as flat extension

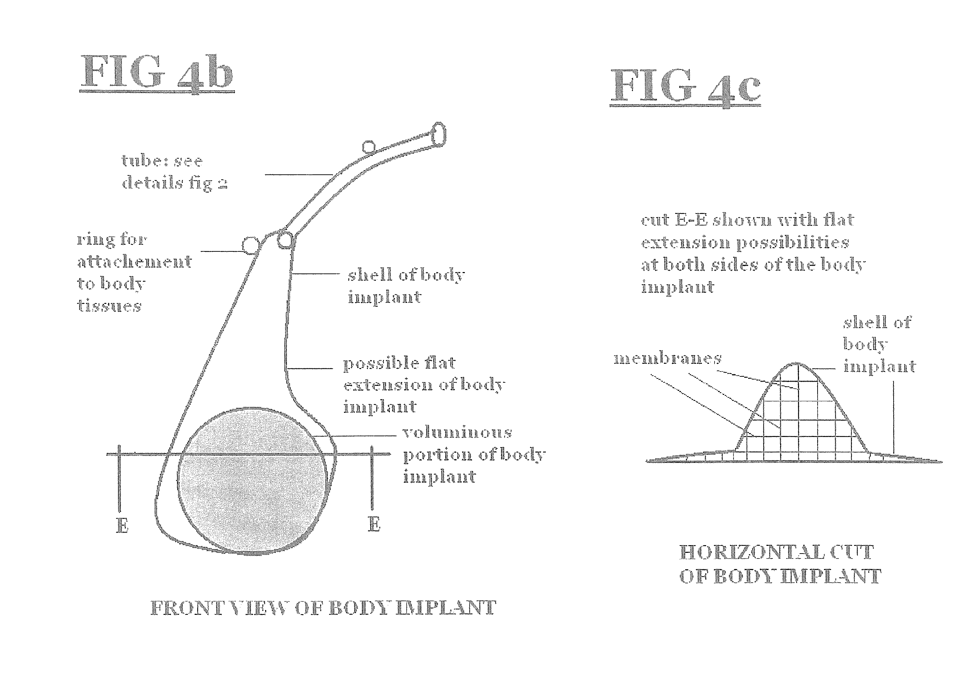

   - FIG 4B: : front view of body implant with principle of flat extension for purpose of stability of the implant being a breast
   - FIG 4C: : sample of horizontal transversal section or cuts F-F at the middle side of body implants being a breast in this case. The section or cuts are showing the vertical membranes as lines in the section or cut, containing vertical shafts as cavities and showing the possible forms of the flat extension of the main external shape of body implant for better stability, those flat extensions can go fully or partially around the main shape of the body implants
   - FIG 4D: : sample of transversal section or cuts D-D at the upper side and E-E at the middle side of body implants being a breast. The section or cuts are showing the vertical membranes as lines in the section or cut, containing vertical shafts as cavities!

### DESCRIPTION:

The invention of a new concept and a new methodology for body implants, based on various principles explained below, will allow multiple and/or repetitive transformation of body parts - as are breasts and penis for instance - which can be fully filled, emptied and refilled by people at home, without surgery! Existing techniques allows for one fill or very limited number of transformation only and are not based on a cavity interconnected volume structure. These new techniques allow for the implants to take a minimum of space when empty to be (nearly) invisible in outer human body or skin, and for surgery purpose having the lowest possible profile for ease of insertion and placement into the body through minimum section or cut or stab wound into the body! These techniques allows for an outside connection via a tube to allow filling and emptying by human being without surgery! The new inside is a grid structure which allows for stability and flexibility of the implant! This grid structure encloses a big number (more then 4 and up to more than 100) of cavities which are all interconnected.

Particularly our implants are made in existing body proof materials (as far as the outer shell concerns) for external contact with the inside of human body tissues (all materials approved by FDA or other local medical authorities for instance as is silicon elastomer). Those external materials can be used to avoid a maximum of body rejection phenomena! The new implant objects can be made with the similar materials as actual used body implants and use the same technical characteristics of the materials. The inside of our implants are filled with a cavity structured material: two types are mentioned as a sample hereby, one being a spongy material, the other one being a grated structure, both having the cavities (more or less) interconnected! The grated structure is formed by thin membranes. The cavities can be as long shafts in vertical, horizontal, oblique or any direction. The cavities can also be as spherical or cubic or any other shape suitable for production techniques and stability purpose. All cavities are interconnected for fluid transfer through porosity or little holes! The implants can have any form or shape that matches all the requests (for example: patient, medical, physical, human body) by adapting our inside grid structure fully to the different implant shapes! This grid structure keeps that external shape when the body implant is inflated or filled up, is a fully compressible structure, that allows the implant to become (nearly totally) flat (except thickness of the thin materials used for the membranes and the shell of the body implant and the tube with in/outlet) when the implant is empty! The inside of the implant can be structured with a grid of fleeces or membranes to keep stability of the required form of the body implant when it is filled up with the liquid. Any form can be given to this outer volume shape through the grid full of membranes that are glued, seamed or fixed by any technique to the inside shell of this outer volume shape.

The cavity structure can be fixed within or without inner shell, itself fixed or not to the inside of the outer shell in relation to the stability, in function of avoiding wrinkles and insuring surgical safety!

The implants will have an inlet/outlet system for filling up and emptying repeatedly with liquids as are (saline) water, (natural) oil with a low to high viscosity, gas like air or any other similar and equivalent acceptable liquids! That inlet/outlet can be a simple (welded, glued, seamed or fixed in any technical most suitable manner) leakage proof connection of the filling tube to the shell of the main body implant; the exit piece of the tube through the human skin can be secured the same way as the existing techniques for any other human body exit system (it maybe like a piercing in the skin). For instance they can use some ring (connected to the tube by welding, gluing, seaming, or any method) in the body skin for keeping the opening accessible at any time when the inflatable body implant has to be filled or emptied! This connection between the filling tube and the exit piece in the skin can be fully secured (see other long term medical applications). This inlet/outlet system can be adapted to any refilling system and its technical requirements. Because this inlet/outlet needs to be connected with a refill drum or tank, with the possibility of quantifying the refill liquid, the connection system has to be safe taking into account all the human body and health aspects.

This inlet/outlet can start at some end of the main part of the body implant which can be extended with a narrowing and/or wide flat transition shaped thin body fitting shape (for instance a flat peer shaped extension) at for instance the top of the main body implant, and extending towards the inlet/outlet system at that place! All similar principles of connection and/or transition pieces between the filling tube and the main body implant are considered in the way of connecting the filling tube through a transition piece that is filled up with a similar grated structure, being in extension or attached to the main body implant! (see possible connection types in drawings) The flat extension can be made in several directions around the main body of the implant for stabilizing it or other purposes.

The thin membranes (giving the structured shape to the body implant and its extensions: see possible sheets profiles in drawings) will allow a flow through of any liquid from one side of the body-implant to another side, meaning left to right, or up to down and vice versa, so that each cavity is connected with the cavity in front of the in/outlet tubes. The cavities between the membranes can be connected with each other through small holes to be placed on adequate locations in the membranes. For instance little holes can be placed near the outer shell at the bottom as well as at the top, and/or left and/or right and/or in the middle! The number and the size of little holes or connections between the cavities in between the membranes is determining the viscosity feeling of the body implant! The fluidity of the used liquid can also influence the viscosity feeling in combination with the transfer holes structure through the membranes. Another system of connection of the cavities between the membranes can be obtained through a certain permeability of the membranes. This system can evolve to spongy materials as far as they can reduce their volume in empty situation without requiring a major pressure from outside. The number of membranes can be functionally determined. Their can be a lot of parallel membranes or fleeces in both directions of the grid, fixed one way or the other (by melting, soldering, gluing, moulding, pouring, etc.) to the outer form or shell of the implant for stability and/or solidity and/or flexibility of the shape into the human body! As mentioned above, the number of membranes influences (slightly) the volume taken into the body when the body implant is emptied, but the number is also related towards the structural and physical characteristics of the body implant as are the viscosity and fluidity feeling when filled up, and as is the shape stability of the body implant! The cavities between the membranes can be filled up properly through one (it can be more, but this will increase technical production complication) filling tube because of cavities of the membranes being connected by either permeable (pervious) membranes or membranes including a number of properly located holes for connecting all cavities to the entrance cavity at the tube connection and for allowing the liquid to be filled in the implant in a balanced way! That number and size of holes in the membranes or the permeability of the membranes can vary to influence the fluid transition in the cavities between the membranes and influencing that way the tactile effects on the implant! The behavior as a flexible mould can be obtained reducing or increasing the permeability or connecting holes, giving a kneadable, malleable, as realistic as possible touching effect! The filling itself will have to be done with the filling tube in more or less vertical position, while the emptying should be made while filling tube is at the same level or lower then the body implant, so that gravity helps emptying the body implant if the system requires it.

Each grid membrane can be designed in straight (rectangular grid) or curved (honeycomb grid) shape or form, all related to the required external and visible body form when inflated. (see sample drawings). The layout of the membranes in the body implant can be made in different ways as samples are listed but not limited to below mentioned samples, and it means that the principle itself covers all variants of those grids mentioned below:
i. A grid of crossing membranes, meaning crossing each other viewed in transversal plan sight section or cut, will be in gratings or as a grid whereby the distance or space in between the membranes is function of the volume taken by this membranes when the body implant is deflated or emptied, and is function as well of the fluidity or viscosity and stability of the inflated body to be obtained,
ii. A grid of crossing membranes, meaning crossing each other under straight angle or any other technically usefully angle!
iii. A grid of parallel membranes having a specific direction within the body implant!
iv. A grid of parallel membranes being straight lined or curved within the body implant, as are honeycombs!

The external materials for the body implants (simultaneously as internal materials being membranes or fleeces) used, which are similar to the ones used for actual implants today, can have some elasticity or stretch for filling some different quantity, this means more or less liquid within a certain range of volume going from for example 50 to 150% easily, 100% being the optimal filling volume! (A filling security valve should be inserted in implant) This stretching of materials will be limited to the main body of the implant, avoiding stretching in the extensions of the body implant.

The body implants can be placed by specialized surgery doctors into its place using the modern techniques and methodology that allow introducing small volumes (our empty implants can be packed in ideal format for surgery) through small diameter of insertion into the body. Once introduced in between the body tissues, the implant can be spread out in place. This can be spread out directly under the skin as well as under or in between other tissues. These implants can be inserted for example in flat condition, in circular or cylindrical format or even filled up or inflated (the traditional way) as per easiest operational requirements for surgery and medical requirements or future results.

The refill flexible tube as per required length from exit of implant or its extension towards the skin exit (being potentially at the nipple or under armpit in case of breast implant) will be executed in same flexible (but not stretchable) materials as the implants with a possibility of remaining flat when no liquid is running through it! As mentioned for example an inlet/outlet end of tube of the body implant can be put in the nipple or the (flat flexible) tube can end up under the armpit in the case of a reinflatable breast implant! The tube doesn't need to be circular or round when seen in its transversal section or cut, but can also have a flat section or any suitable section.

The refill flexible tube as well as every side of the implants can have one or more rings attached (by welding, gluing or seamed) or integrated to the body implant or the tube or the flat extension of the implant for suturing the tube or implant to body tissues for fixing the implant avoiding it to undergo the gravity or other effects which can displace the implant! The used material can be the existing material used for such function!

The plug or valve can be located at the inner side of the tube where it is connected to the body implant. This plug or stop or valve will keep the liquid in to the body implant. When the stop or plug or valve will be placed in the main structure of the body implant where the filling tube is starting, we avoid having liquid in the filling tube or pipe which is concerning its visibility better on certain places in the human body. The plug or valve can also be located at the outlet side of the tube connected to the body implant. This plug or stop or valve will keep the liquid into the body implant and its filling tube, but is less recommended if the tube is long, because it is filed up with liquid and can be visible in relation to its eventually round shape. This is less the case with a flat shaped tube. This plug or valve or closing system fitting in the in/outlet hole of the inside end (or in the other case near the outside end) of the filling tube, which end could be of conical, cylindrical or every suitable shape, has to be waterproof or liquid-proof when closing the body implant filled up with liquid! This closing system can be made secured with a special pressure valve for avoiding overfilling! This plug or valve can be opened for instance by turning and/or pushing a flat (or whatever suitable shape) handle at the end of a wire commando. The plug can work as a compress and expansion system, a screw system or any leakage proof system! The system will need to be adapted to a leakage proof refill connection from a filler drum! We can even consider the filling through needles and syringe system with required volume to fill and of course a closing system for the needle hole!

This plug can be linked to the little (preferably flat) handle at the external end of the tube (at exit place in the human body) with a thread, wire or fibre in any acceptable and resistant material as for instance are composite materials, metals or any material not damaging the inside of the tube, since the wire is located inside the tube. The wired commando will be stiff enough for commanding the plug. This wired commando can be of low (= stiff) or high torsion flexibility related to the plug closing system!

The tube can but does not need to contain a shaft for the wired commando. This shaft can have different reasons: place fixing in the tube, wear and tear of thread in the tube, insulating grease protection, etc.

The handle will be located at the external end of the flat or round tube, near the body exit place of the tube! The handle at the exit place through the skin can have an "entering stop system" to avoid full disappearance or inaccessibility of the handle into the tube. This handle can have the turning and/or pulling/pushing function for instance.

All the features mentioned above can be combined in any way to realise the refillable or re-inflatable implants.

## Claims

1. A multi refillable and/or re-inflatable, flexible and/or extensible implant made in soft materials and based on a new concept and a new methodology for body implants, with various principles explained further, to allow multiple and/or repetitive transformation of body parts - as are breasts and penis for instance - which can be fully filled, emptied and refilled by people at home, without surgery and whereby the technical method allows multiple and repetitive transformation through an internal build up of a 2 or 3-dimensional grated membrane structure inside the shell of the implant, forming a multi cavity structure and whereby this grated membrane structure contains a fluid transition system for filling and emptying through permeability or small holes properly placed in the membranes to allow cavity connections, which bring the cavities of that grated structure in connection to a in/outlet system.

2. An implant according to claim 1, **characterised in that** the inside of the implants are filled with a cavity structured material which is composed as one or more grated membrane structures, having the cavities more or less interconnected, and which can be like a spongy material, and whereby the grated structure formed by thin membranes, that enclose the cavities that can be as long shafts in vertical, horizontal, oblique or any direction in the case of 2-dimensional grated structure or the cavities that can also be as spherical or cubic or any other shape in the case of 3-dimensional structure, to finally constitute any form or shape that matches the requests of the required implant through adapting the inside grid structure fully to the different implant shapes, considering that this grid structure keeps that external shape fixed and stable but flexible when the body implant is inflated or filled up and considering that this grid structure is a fully compressible structure, which allows the implant to become flat when the implant is empty, because those membranes are made out of very flexible, thin multi-foldable materials.

3. An implant according to claim 2, **characterised in that** the cavity structure can be fixed within or without inner shell, fixed or not to the inside of the outer shell in relation to the production techniques.

4. An implant according to claim 1, **characterised in that** the implant will have an inlet/outlet system for filling up and emptying repeatedly with liquids as are (saline) water, (natural) oil with a low to high viscosity, gas like air or any other similar and equivalent acceptable liquids, whereby that inlet/outlet consist of a valve or plug that can open and close multiple times, a connection tube or pipe from the implant to the skin exit in the human body, wherever it is located, and a handle for opening and closing the refilling inlet/outlet system.

5. An implant according to claim 1, **characterised in that** the main part of the body implant has 1 or more narrowing and/or wide flat transition shaped thin body extensions at the top or every other side of the main body implant, whereby this extension(s) can function as transition piece towards the inlet/outlet system starting at that place and/or those extensions can also help stabilizing the body implant into its place.

6. An implant according to claim 1 or 2, **characterised in that** the grid structure of the thin membranes will allow a flow through of any liquid from one side of the body-implant to another side, meaning left to right, or up to down and vice versa, so that each cavity can be finally connected with the cavity in front of the in/outlet tube, and all cavities are interconnected for fluid transfer through small holes to be placed on adequate locations in the membranes, taking into account that the number and the size (porosity if very little) of little holes or connections between the cavities in between the membranes are determining the viscosity feeling of the body implant, in combination with the fluidity of the used liquid that influences the viscosity feeling in combination with these transfer holes structure through the membranes, whereby the holes are allowing the liquid to be filled in the implant in a balanced way, through one or more filling tubes, knowing that when the size of the holes through the membranes is reduced under a certain size, then we obtain a certain permeability of the membranes replacing the holes.

7. An implant according to claim 1 or 2, **characterised in that** each membrane grid is designed in straight (rectangular grid for instance) or curved (honeycomb grid for instance) shape or any other 3-dimensional grated structure whereby all designs are giving, when inflated, the required external and visible body form independently of the layout of the membranes in the body implant that can be made as crossing membranes under all different angles, as well 2 as 3-dimensionally.

8. An implant according to claim 1 or 2, **characterised in that** the external materials for the body implants simultaneously as internal materials being membranes or fleeces used, have some elasticity or stretch for filling some different quantity, meaning that more or less liquid can be filled within a certain range of volume going from, but not limited to, for instance 50 to 150% easily, 100% being the optimal filling volume for the materials used.

9. An implant according to claim 1 or 2, **characterised in that** the empty body implants are arranged to be placed by specialized surgery doctors into its place using the modern techniques and methodology that allow introducing small volumes through small diameter of insertion into to body, because the empty or flat implants can be packed in ideal circular or cylindrical or any small diameter format for surgery introduction, so that once introduced in between the body tissues, the implant, its eventual extension, and the in/outlet system can be spread out in place, being it directly under the skin as well as under or in between other tissues.

10. An implant according to claim 1 or 4, **characterised in that** the refill flexible tube as per required length from exit of implant or its extension towards the skin exit is executed in similar flexible materials (not requiring any stretch characteristics) as the implants with a possibility of remaining flat when no liquid is running through it, considering that the tube doesn't need to be circular or round when seen in its transversal section or cut, but can also have a flat half oval section or any suitable section, and considering that the tube system will need to be adapted at its exit side to a -leakage proof- refill connection from a filler drum.

11. An implant according to any one of the claims 1, 4 and 5, **characterised in that** the refill flexible tube as well as every side of the implants can have one or more rings attached or for suturing the tube and/or implant to body tissues for purpose of fixing the implant avoiding displacement when it undergoes the gravity or other body effects which can displace the implant.

12. An implant according to claim 1 or 4, **characterised in that** there is a plug or valve that can be located at the inlet or outlet side of the tube connected to the body implant or its extension, which will keep the liquid in to the body implant, considering that being placed at the side of the main structure of the body implant where the filling tube is starting, it avoids having liquid in the filling tube or pipe being better concerning its visibility on certain places in the human body, or being located at the outlet side of the tube connected to the body implant, keeping the liquid into the body implant and its filling tube too, being less recommended if the tube is long and with a larger section or diameter because it is filed up with liquid and can be visible, being less the case with a flat shaped tube, and finally considering that this valve or closing system, that fits in the in/outlet hole of the filling tube, has to secure waterproofing when closing the body implant filled up with liquid.

13. An implant according to claim 1 and 12, **characterised in that** this plug or valve can be linked to a little handle at the external end of the tube with a thread, wire or fibre in any acceptable and resistant material as any material not damaging the inside of the tube, since the wire is located inside the tube, whereby the wire can also be a security and emergency solution to open the valve when it is commanded wireless.

14. An implant according to any one of the claims 1, 10 and 13, **characterised in that** the tube contains a shaft for the wired commando.

15. An implant according to any one of the claims 1, 4, 10, 12, and 13, **characterised in that**, this handle will be located at the external end of the tube in the case of a manual command, near the body exit place of the tube, or the handle can also be built on the valve itself in the case of wireless commando having the function of opening and/or closing the valve.

16. According to all preceding claims, any new combination of above technical applications and structures to realize the refillable or re-inflatable implants is part of this patent.
